(19) **Europäisches Patentamt European Patent Office Office européen des brevets**

(11) **EP 4 305 488 B1**

(12) **EUROPEAN PATENT SPECIFICATION**

(45) Date of publication and mention
of the grant of the patent:
**15.10.2025 Bulletin 2025/42**

(21) Application number: **22714044.9**

(22) Date of filing: **09.03.2022**

(51) International Patent Classification (IPC):
**G02C 7/02** (2006.01)

(52) Cooperative Patent Classification (CPC):
**A61F 2/16; G02C 7/028;** G02C 7/044

(86) International application number:
**PCT/US2022/019463**

(87) International publication number:
**WO 2022/192339 (15.09.2022 Gazette 2022/37)**

(54) **INTRAOCULAR LENS PROVIDING EXTENDED DEPTH OF FOCUS**

INTRAOKULARE LINSE MIT ERWEITERTER SCHÄRFENTIEFE

LENTILLE INTRAOCULAIRE AVEC UNE PROFONDEUR DE CHAMP ÉTENDUE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **09.03.2021 US 202163158414 P**

(43) Date of publication of application:
**17.01.2024 Bulletin 2024/03**

(73) Proprietor: **TATVUM LLC
Irvine, CA 92604 (US)**

(72) Inventor: **TIWARI, Nivedan
Irvine, California 92604 (US)**

(74) Representative: **Vossius & Partner
Patentanwälte Rechtsanwälte mbB
Siebertstrasse 3
81675 München (DE)**

(56) References cited:
**EP-A1- 0 938 016     EP-B1- 2 403 429
US-A1- 2009 270 984     US-A1- 2017 276 961**

**US-A1- 2018 243 082     US-A1- 2020 253 719
US-A1- 2020 315 850     US-A1- 2021 055 573
US-B1- 6 457 826**

- YANG YABO ET AL: "Pupil location under mesopic, photopic, and pharmacologically dilated conditions", INVESTIGATIVE OPHTHALMOLOGY & VISUAL SCIENCE, ASSOCIATION FOR RESEARCH IN VISION AND OPHTHALMOLOGY, US, vol. 43, no. 7, 1 July 2002 (2002-07-01), pages 2508 - 2512, XP002537709, ISSN: 0146-0404
- PAZO ERIC E. ET AL: "Pupil influence on the quality of vision in rotationally asymmetric multifocal IOLs with surface-embedded near segment", JOURNAL CATARACT AND REFRACTIVE SURGERY., vol. 43, no. 11, 1 November 2017 (2017-11-01), US, pages 1420 - 1429, XP055929527, ISSN: 0886-3350, DOI: 10.1016/j.jcrs.2017.08.013

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 4 305 488 B1

**Description**

FIELD

Intraocular lenses, and in particular intraocular lenses providing an extended depth of focus.

BACKGROUND

The use of intraocular lenses (IOLs) to replace natural crystalline lenses that have become opaque due to cataracts is well established. IOLs typically comprise an optic and haptics for positioning the optic within an eye.

Monofocal IOLs are the most commonly implanted type of IOLs. Monofocal lenses are selected to provide only distance vision, leaving objects at intermediate and near distance out of focus for the user, and necessitating the use of eyeglasses.

Multifocal IOLs provide a range of vision including distance, intermediate and/or near vision regions by generating focal points corresponding to each vision region. Conventional multifocal lenses typically fit into one of two classes - refractive or diffractive.

Refractive multifocal IOLs have an optic that is divided into multiple refractive power regions with light from a particular region being directed, using refractive power, to only one corresponding lens focus. Typically, refractive multifocal lenses form two or more foci to provide far, intermediate and/or near vision. Such lenses have a region providing base power (typically selected to provide distance vision) with the remaining region(s) introducing add power to provide intermediate and/or near vision.

Like refractive multifocal lenses, diffractive multifocal lenses form two or more foci to provide far, intermediate and/or near vision; however, diffractive multifocal lenses use a diffractive element comprising radial zones to direct light to the foci. In diffractive multifocal lenses, the radial edges that separate the zones are chosen to achieve particular optical powers associated with the foci. Typically, a diffractive lens has an underlying refractive power providing a base power selected to provide distance vision, and the diffractive elements(s) introduce add power to provide intermediate and/or near vision. Both diffractive multifocal and refractive multifocal lens techniques result in IOLs having a depth of focus with distinct foci where vision is sharp, and regions of poorer vision between the foci.

A well-known example of a figure of merit for measuring the performance of visual systems at a given location is known as a Modulation Transfer Function (commonly referred to as an "MTF"). An MTF of an optical system is a measure of the proportion of contrast of an input object that the optical system is able to maintain when an image of the object is produced. An MTF can be measured as a function of spatial frequency (e.g., line pairs per millimeter (mm) at the retina). Generally, the MTF values for a given optical system decrease with an increase in the spatial frequency. For a given spatial frequency, an MTF curve can be plotted as a function of distance from a lens. An MTF measured as a function of distance from a lens is referred to as a through-focus MTF.

For a given spatial frequency, each of one or more foci of an IOL manifests itself in a through-focus MTF plot as a peak in the MTF curve. Monofocal lenses have a single peak in MTF, whereas multifocal lenses have two or more peaks in MTF (e.g., corresponding to near, intermediate and/or far focus). Surrounding the peak(s) are regions of lower MTF.

Some lenses are designed to spread light energy from one or more of the peaks to a region that would otherwise have a lower MTF value, such that a region that would otherwise not support vision is able to provide acceptable vision. Such lenses are referred to as extended depth of focus (EDOF) lenses.

IOL design techniques to provide EDOF capabilities include: a) providing an IOL with a central refractive add zone; b) providing an IOL with relatively high-magnitude positive or negative spherical aberration; and c) providing an underlying refractive IOL with a relatively low-power add, diffractive profile. These EDOF techniques have provided visual quality and patient satisfaction that has varied individual-to-individual, with wearers experiencing different levels of visual quality across the depth of focus, as well as experiencing varying degrees of dysphotopsia (e.g., halos or ghosting). Examples of IOL that provide EDOF are disclosed in US 2020/253719 A1

EDOF IOLs that result in less individual-to-individual variation are needed.

SUMMARY

The inventors have determined that individual-to-individual variation in EDOF IOL performance results from differences in pupillary size and pupillary response characteristics, differences in corneal aberrations (which could aid or negate the extension in depth of focus), and/or differences in sensitivity to scattered light. Such variations tend to decrease wearer satisfaction as measured for a given population of wearers.

Aspects of the present invention are directed to IOLs in which feature(s) of the lens that generate an extended depth of focus (EDOF) are located on the IOL within a radial position corresponding to the pupil of the user's eye for selected lighting conditions. Such a design reduces the likelihood that a person in a target population will experience dysphotopsia (e.g., halos or ghosting). In such designs, the features are located within a radial position corresponding to photopic vision conditions, for example, such that the base power (providing distance vision) is present over at least 30% of the radially outward portion of the pupil of the user's eye under photopic vision conditions. Such designs allow an eye to achieve adequate distance resolution as well as extended depth of focus performance.

By limiting the EDOF feature(s) to an inner portion of the photopic pupil, individual-to-individual variations within a population are limited, in part, because the magnitude of aberrations present in each patient within the population tends to be less toward the inner portion of the pupil resulting in less variations in aberrations across the population. Additionally, maintaining the EDOF features to within the inner portion of the photopic pupil limits the impact of pupil size and pupillary response. Furthermore, such a design reduces or eliminates the possibility of pupil dilatation exposing additional EDOF features under low light conditions. Additionally, by avoiding the presence of EDOF features in the relatively large area of the pupil that is used for night vision (scotopic vision), the likelihood of introducing light scattering from EDOF features at night, when wearers are particularly susceptible to dysphotopsia.

It will be understood that IOLs are typically not customized to a given user's eyes, and that the radial location of the feature(s) may be selected statistically, based on pupil measurements of a population (i.e., the EDOF features are located such that base power is present over at least 30% of the radially outward portion of a pupil's eye under photopic vision conditions for at least 60% of a selected population or at least about 70% of a selected population. For example, to achieve the desired result in a given population, the features may be located within a region within a radius of 0.7 mm or 1.2 mm or 1.4 mm about the optical axis.

Although a lens may be surgically implanted at a location displaced from the wearer's pupil (e.g., in the posterior chamber, such as in the capsular bag), it is assumed herein that the radial pupil dimension is equal to the radial dimension on the lens.

An aspect of the invention is as defined in claim 1.

According to the claimed invention, the first region is increasing in curvature as a function of increasing radial position from the optical axis to the outer edge of the first region; and the second region is decreasing in curvature as a function of increasing radial position from the outer edge to achieve the powers less than the base power, and then increasing in curvature as a function of radial position to achieve the base power.

In some embodiments, the sagittal surface profile may be specified by an equation z(r), where

$$z(r) = r^2 \left\{ R + \sqrt{R^2 - (1+c)r^2} \right\}^{-1} + \propto_m (r) * r^m$$

and where

R is the surface radius of curvature,
c is the surface conic constant, and m is 4 or higher (i.e., $\alpha_m(r)r^m$ is comprised of one or more terms where m is an integer of 4 or higher).

In some embodiments, m = 4 (i.e., $\alpha_m(r)r^m$ is comprised of a single term of where m = 4).

According to the claimed invention, the base power is maintained in the second region over a radial distance of at least 0.3 mm. In other such embodiments, substantially the base power is maintained in the second region over a radial distance of at least 0.6 mm.

In some embodiments, $\alpha(r)$ varies exponentially as a function of radial distance.

$$\text{For example, } \propto (r) = \begin{cases} \text{r} \leq r_{lim}; & \propto_1 + 2(\propto_2 - \propto_1)\{(1 + e^{-Ar})^{-1} - 0.5\} \\ \text{r} > r_{lim}; & \text{the coefficeint} \propto (r) \text{ is defined to attain} \\ & \text{a value } \propto_1 \end{cases}$$

where r is the radial distance from the lens optical axis,
$\alpha_1$, $\alpha_2$ and A are constants defining variation of the fourth order coefficient as a function of r,
$r_{lim}$ is the location of the outer edge of the first region and defines a radial transition location for the fourth order coefficient ($\alpha(r)$).

In some embodiments, for r > $r_{lim}$

$$\propto (r) = \propto_1$$

whereby $\alpha(r)$ attains value $\alpha_1$ at $r_{lim}$.

In other embodiments, for $r > r_{lim}$

$$\propto (r) = \propto_2 + 2(\propto_1 - \propto_2)\left\{\left(1 + e^{-B(r-r_{lim})}\right)^{-1} - 0.5\right\}$$

where, B is a constant, whereby $\alpha(r)$ attains value $\alpha_1$ gradually for values of r greater than $r_{lim}$.

In some embodiments, z(r) further comprises a term $z_{shift}$ where

$$z_{shift} = \begin{cases} r \leq r_{lim}; \ 0 \\ r > r_{lim}; \ \left(\propto_{lim}(r_{lim})^4 - \propto_1(r_{lim})^4\right)\left(2\left(\left(1 + e^{-B(r-r_{lim})}\right)^{-1} - 0.5\right)\right) \end{cases}$$

In some embodiments, the sagittal surface profile can be specified by an equation z(r), where

$$z(r) = r^2\left\{R + \sqrt{R^2 - (1+c)r^2}\right\}^{-1} + \text{EDOF features sagittal profile term}$$

and,
wherein the EDOF features sagittal profile term is specified using a polynomial expression.

In some embodiments, the polynomial expression approximates an exponential variation. For example, the polynomial expression may be of a form $\sum_{n=1}^{m} C_{2n} r^{2n}$ .

The polynomial expression may be of 24th order or greater, whereby $m \geq 12$ (i.e., terms where m is less than 12 are not present).

In some embodiments, the surface is an anterior surface or a posterior surface, and the non-increasing curvature portion is characterized by a discontinuity located at the outer edge, such that the outer edge and the location of minimum lens power are substantially radially coincident.

In some embodiments, the surface is an anterior surface or a posterior surface, and the non-increasing curvature portion varies smoothly as a function of radius such that the distance from the outer edge of the first region to a radial location of a minimum lens power is less than 0.4 mm

The lens may be monofocal.

In some embodiments, the sagittal surface profile is further defined by a diffractive profile superimposed on the surface.

The diffractive profile may be configured to produce a depth of focus having a first peak in MTF and a second peak in MTF, and the EDOF features increase the MTF between the first peak and the second peak, where the MTF is specified using the ISO 1 model eye, and where the MTF is specified for a spatial frequency of 50 lp/mm at the retina, at 546 nm light, for a 3 mm diameter pupil, when the lens is immersed in aqueous humor having a refractive index of 1.336 at 546 nm.

The EDOF features may increase the MTF only between the first peak and the second peak. In some embodiments, the diffractive profile is a bifocal diffractive profile. In some embodiments, the maximum MTF value is equal to or greater than 0.35.

In some embodiments, the first peak in the MTF and the second peak are separated by about 2.5 diopters, and the EDOF features provides a depth of focus continuous with the first peak that maintains an MTF of 0.15 or greater for at least about 1.25 diopters, in the myopic direction from the first peak.

Another aspect of the present invention is defined in claim 10.

In some embodiments, the first region is increasing in power as a function of increasing radial position from the optical axis to the outer edge of the first region; and the second region is decreasing in power as a function of increasing radial position from the outer edge to achieve the minimum lens power, and then increasing in curvature as a function of radial position to achieve the base power.

In some embodiments, the increase in power across the first region is in the range of 0.5 to 5.0 diopters. According to the claimed invention, the lens achieves a perceived depth of focus in the range of 1.0 to 1.5 diopters.

The lens may be monofocal.

In some embodiments, there is a step in power at the outer edge.

In some embodiments, the power between the outer edge and the minimum lens power is smoothly varying and the decrease in power between the outer edge and the minimum lens power occurs over a radial distance of less than 0.4 mm. In other embodiments, the lens comprises an anterior surface and a posterior surface, wherein the power profile is achieved by a change in surface curvature in at least one of the anterior surface and the posterior surface, and wherein the anterior surface or the posterior surface has a step in power at the outer edge of the first region.

In some embodiments, the lens comprises an anterior surface and a posterior surface, wherein the power profile is achieved by a change in surface curvature in at least one of the anterior surface and the posterior surface, and wherein neither the anterior surface nor the posterior surface has a step in power at the outer edge of the first region.

In some embodiments, the lens comprises an anterior surface and a posterior surface, wherein the power profile is achieved by a change in surface curvature in at least one of the anterior surface and the posterior surface, and wherein the non-increasing portion of the second region is characterized by a power as a function of radius that is smoothly varying such that the distance from the outer edge of the first region to a radial location of the minimum lens power is less than 0.4 mm.

In some embodiments, the lens is a gradient index lens having an index of refraction that varies radially to provide the power profile.

According to the claimed invention, substantially the base power is maintained in the second region over a radial distance of at least 0.3 mm. In some embodiments, substantially the base power is maintained in the second region over a radial distance of at least 0.6 mm.

In some embodiments, the lens further comprises a surface having a diffractive profile superimposed thereon.

In some embodiments, the diffractive profile may be configured to produce a depth of focus having a first peak in MTF and a second peak in MTF, and wherein the power profile increases the MTF between the first peak and the second peak, where the MTF is specified using the ISO 1 model eye, and where the MTF is specified for a spatial frequency of 50 lp/mm at the retina, at 546 nm light, for a 3 mm diameter pupil, when the lens is immersed in aqueous humor having a refractive index of 1.336 at 546 nm.

The power profile may increase the MTF only between the first peak and the second peak. The diffractive profile may be a bifocal diffractive profile.

In some embodiments, the maximum MTF value is equal to or greater than 0.35. In some embodiments, the first peak in the MTF and the second peak are separated by about 2.5 diopters, and the power profile provides a depth of focus continuous with the first peak that maintains an MTF of 0.15 or greater for at least about 1.25 diopters, in the myopic direction from the first peak.

Yet another aspect of the disclosure but not of the claimed invention is directed to an intraocular lens having an optical axis and providing an extended depth of focus using refractive feature(s) within a 1.4 mm radial distance of the optical axis to generate a through-focus MTF characterized by a first peak having an absolute maximum MTF value in excess of 0.35 (e.g., between 0.35 to 0.85 or between 0.5 to 0.85). The lens has a region continuous with the first peak maintaining an MTF value of greater than 0.15 (e.g., between 0.15 to 0.6) to achieve a depth of focus of at least about 1.25 diopters extending in the myopic direction from the first peak. The MTF is specified using the ISO 1 model eye; and the MTF is specified for a spatial frequency of 50 lp/mm at the retina, at 546 nm light, for a 3 mm diameter pupil, when the lens is immersed in aqueous humor having a refractive index of 1.336 at 546 nm.

In some embodiments, the lens has only a single peak in the through-focus MTF, whereby the lens is monofocal.

In some embodiments, the maximum through-focus MTF value is equal to greater than 0.5.

In some embodiments, the refractive features are within a 1.2 mm radial distance from the optical axis.

In some embodiments, the lens is a gradient index lens having an index of refraction that varies radially, and wherein the refractive features are GRIN features.

The lens may further comprise a surface having a diffractive profile superimposed thereon. The diffractive profile may be configured to produce a second peak in the through-focus MTF, and wherein the region continuous with the first peak extends between the first peak and the second peak. In some embodiments, the refractive features increase the MTF only between the first peak and the second peak.

As used herein, the term "sagittal surface profile" refers to specification of the shape of a surface of a lens surface using, for each location on the surface, a distance from a plane perpendicular to the optical axis and intersecting the surface at the optical axis, the distances measured parallel to the optical axis. A sagittal surface profile is commonly referred to as a surface SAG or, simply, a SAG. If a lens is rotationally symmetric, a single, sagittal surface profile extending from the optical axis to the edge of the optical zone of a lens is sufficient to fully specify the shape of the surface. Although the present invention does not exclude non-rotationally symmetric lenses, in the discussion below, lenses are assumed to be rotationally symmetric unless otherwise stated.

As used herein the term "base power" refers to the nominal power of a lens, and with an IOL means the power corresponding to distance vision, unless otherwise specified. As used herein, the term "base-power sagittal surface profile" refers to a sagittal surface profile of a surface that, in combination with an opposing surface of a lens, provides base power. As used herein, the term "curvatures corresponding to a base-power sagittal surface profile" refers to surface shape that, in combination with an opposing surface of a lens, provides base power. As is known in the art, to construct a lens having a single power (such as the base power) one or both surfaces of the lens may vary in curvature as a function of radial position (i.e., be aspheric), for example to reduce or eliminate spherical aberration.

These and other aspects of the present invention will become apparent upon a review of the following detailed description and the claims appended thereto.

BRIEF DESCRIPTION OF THE DRAWINGS

FIG. 1A is a schematic plan view of an example of extended depth of focus (EDOF) intraocular lens (IOL) according to aspects of the present invention;

FIG. 1B is a schematic cross-sectional side view of the EDOF IOL of FIG. 1A taken along lines 1B - 1B;

FIG. 2A is an example of a sagittal surface profile of a surface of an EDOF lens according to aspects of the invention;

FIG. 2B is a sagittal surface profile of the EDOF features of a surface of an EDOF lens of the lens as shown in FIG. 2A (i.e., aspects of the surface that are determined by the conventional conic term are omitted);

FIG. 3 is a graphical illustration of a power profile of a 20-diopter lens including the lens surface illustrated in FIG. 2A;

FIG. 4 is a graphical illustration of a through-focus MTF for the 20-diopter lens discussed with reference to FIG. 3;

FIG. 5 is second example of a sagittal surface profile of a surface of an EDOF lens according to aspects of the invention;

FIG. 6A is a sagittal surface profile of EDOF features of a surface of an EDOF lens (i.e., the conic term is omitted) in which the EDOF features are specified using a polynomial approximation of the EDOF features of the surface shown in FIG. 2B;

FIG. 6B is a graphical illustration of a through-focus MTF for a 20-diopter lens including the surface using a polynomial definition of the EDOF features as shown in FIG. 6A;

FIG. 7 illustrates an example of a sagittal surface profile of a surface of a refractive-diffractive EDOF lens according to aspects of the present invention; and

FIG. 8 a graphical illustration a through-focus MTF for a 20-diopter lens having a surface as shown in FIG. 7.

DETAILED DESCRIPTION

Aspects of the invention will be further discussed with reference to the following specific examples. It is understood that these examples are given by way of illustration and are not meant to limit the claimed inventions to any particular example.

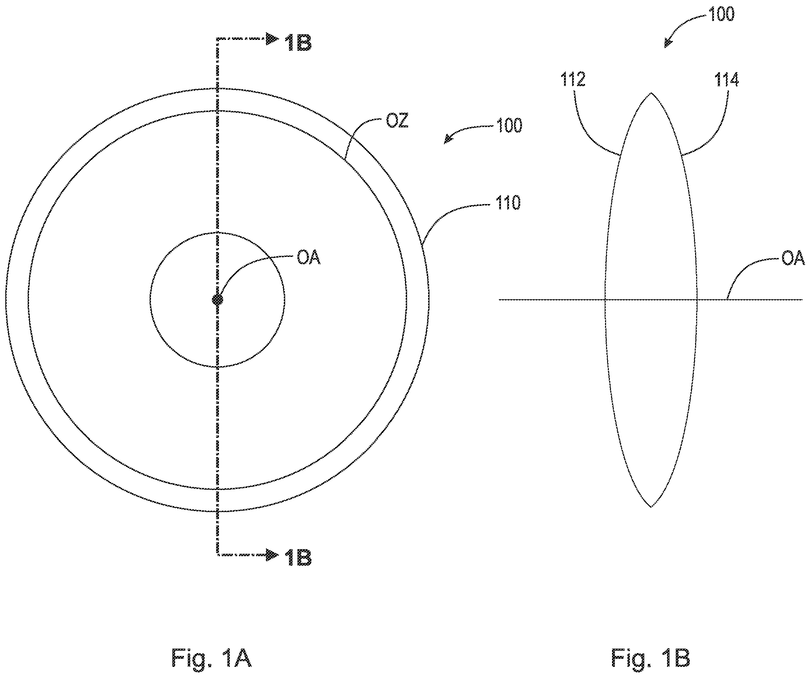

FIGs. 1A and 1B are schematic plan and cross-sectional side view illustrations, respectively, of an example of an EDOF intraocular lens 100 having a base power for achieving distance vision and an extended depth of focus according to aspects of the present invention. As indicated below, IOL 100 has features that generate an extended depth of focus (EDOF) located within a radial perimeter corresponding to the pupil of the user's eye for photopic conditions.

IOL 100 comprises an optic 110 having an optical axis OA, and an anterior surface 112 and a posterior surface 114 both extending over an optical zone OZ. IOLs, such as IOL 100, typically have two or more haptics (not shown) to position the optic within an eye, although in some embodiments a single haptic may be present. Typically, lens 100 is rotationally symmetric, although, some embodiments may be rotationally asymmetric (e.g., toric).

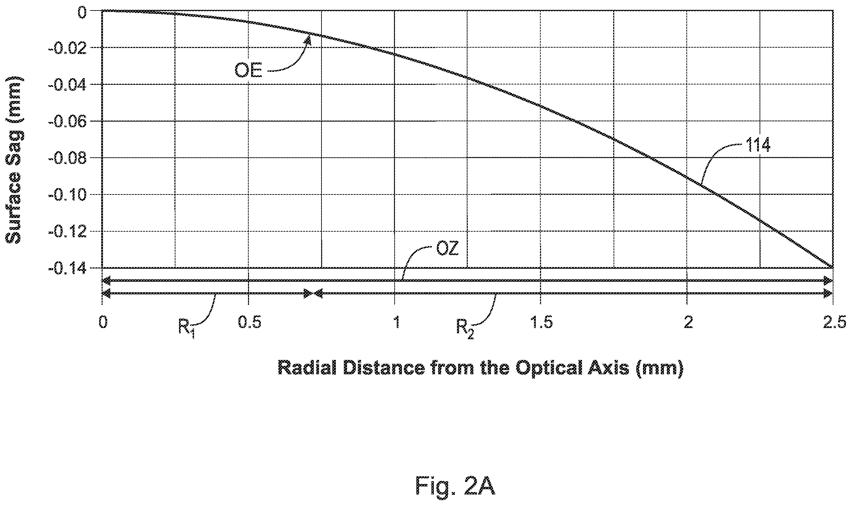

FIG. 2A is an example of a sagittal surface profile of a surface 114 of EDOF lens 100 (shown in FIG. 1) according to aspects of the invention. In the illustrated embodiment, the posterior surface 114 has a sagittal surface profile 200 defined in-part by EDOF features having a first region $R_1$ that is increasing in curvature as a function of increasing radial position from optical axis AO to an outer edge OE of the first region $R_1$. The curvatures in the first region become greater than curvatures corresponding to a base-power sagittal surface profile as radial position is increased toward outer edge OE (i.e., the lens achieves a power greater than the base power in at least a portion of the first region). Although, in the illustrated embodiment, the first region has a monotonically increasing curvature, the first region may have one or more regions of uniform curvature and otherwise increase; that is, the first region may have a non-decreasing curvature. As set forth below, a sagittal surface profile may be further defined by a conventional conic term and/or a diffractive element.

The sagittal surface profile of posterior surface 114 has a second region $R_2$ extending radially outward from outer edge OE, the sagittal surface profile in the second region is decreasing in curvature (as a function of radial position) from outer edge OE. The curvatures become less than curvatures corresponding to the base power profile (i.e., the lens achieves a power less than the base power in a portion of the second region). The curvatures in the second region are then non-decreasing (as a function of radial position) back to curvatures corresponding to the base power profile. Although, in the illustrated embodiment, the second region is decreasing in curvature and then increasing in curvature, the second region may have one or more regions of uniform curvature; that is, the second region may have a non-increasing curvature and then a non-decreasing curvature to achieve base power.

The curvature across outer edge OE (i.e., extending from a location within the first region to a location within the second region) may vary continuously, first increasing in first region $R_1$ and then decreasing in second region $R_2$; alternatively, there may be a discontinuity in curvature at outer edge OE. Additionally, the sagittal surface profile may be continuous across outer edge OE or may have a step in height.

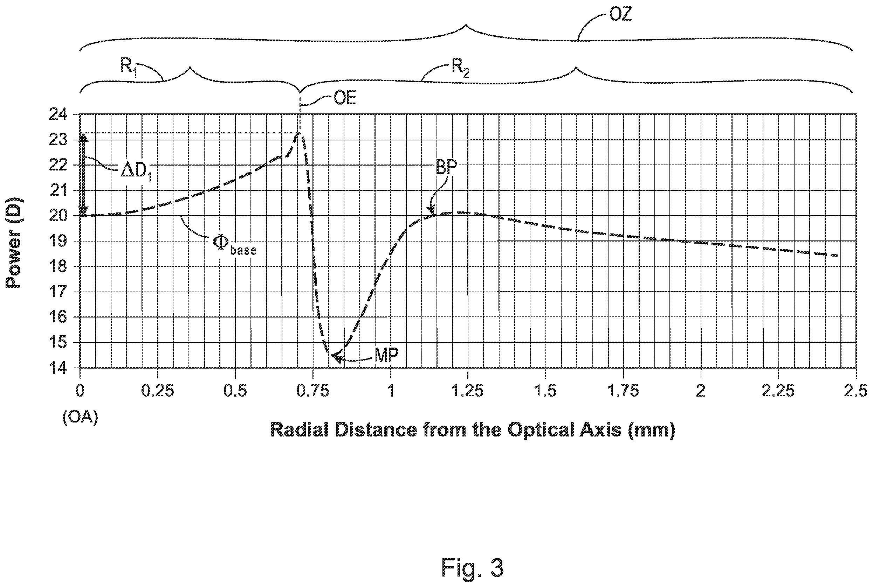

For radial positions outward to where the base power is achieved (i.e., location BP shown in FIG. 3), second region $R_2$ has a sagittal surface profile to maintain substantially the base power profile present over at least 30% of the radial distance corresponding to the pupil of the user's eye for photopic vision conditions. It will be appreciated that providing the base power over the outer portion of the photopic pupil (i.e., limiting the EDOF features to an inner portion of the photopic pupil) will decrease individual-to-individual variation in EDOF IOL performance and improve wearer satisfaction for a given population of wearers. For example, by locating the features of the lens that generate an extended depth of focus within a radial perimeter corresponding to the pupil of the user's eye for selected lighting conditions, the likelihood that a person in a target population will experience dysphotopsia (e.g., halos or ghosting) is also decreased. It will be appreciated that the optical zone extends further from the optical axis than a distance corresponding to the pupil of the user's eye for photopic vision conditions to accommodate vision during lower lighting conditions than photopic conditions. For example, the optical zone may extend to a radial distance from the optical axis to about 2.0 mm to 2.5 mm or about 3.0 mm. As stated above, according to aspects of the present invention, it is desirable that EDOF features not extend into a portion of a lens that is used for low light conditions (i.e., beyond the portion used for photopic vision).

For a given population, the pupil of a person's eye under photopic vision conditions, will typically be in the range 1.0 - 2.0 mm in radius. It will be appreciated that if, as set forth above, the base power profile is present over at least 30% of the radial distance corresponding to the pupil of the user's eye for photopic vision conditions (e.g., 0.3 - 0.6 mm), the EDOF features will be limited to 70% or less of the radial distance corresponding to the pupil of the user's eye for photopic vision conditions.

It is to be appreciated that, although the lens achieves the base power, the power is maintained at the base power or at substantially the base power (i.e., within about +/- 10% of base power), for example, if compensation for spherical aberration of a user's eye is provided in the lens prescription.

Lenses having a surface as described above also comprise an opposing surface (i.e., as shown in FIG. 1B). The opposing surface may be convex or concave. The curvature of the opposing surface may be constant as a function of radial position. Alternatively, the curvature may be monotonically increasing or decreasing as function of radial position, from the optical axis to the edge of the lens. For example, to eliminate or reduce spherical aberration caused by the opposing surface or to compensate for spherical aberration of a user's eye, as is known in the art, (e.g., a convex surface may have a curvature that is monotonically decreasing as a function of radial location).

For the present example and all examples herein, the lens material is hydrophobic acrylic having a refractive index of 1.5332 at 546 nm. Although hydrophobic acrylic lenses are used in the examples, other materials may be used to achieve designs according to aspects of the present invention, such as hydrophilic acrylic, polymethyl-methacrylate (PMMA) or silicone. Lenses having EDOF features as described herein can be manufactured using any suitable technique, such as molding or machining.

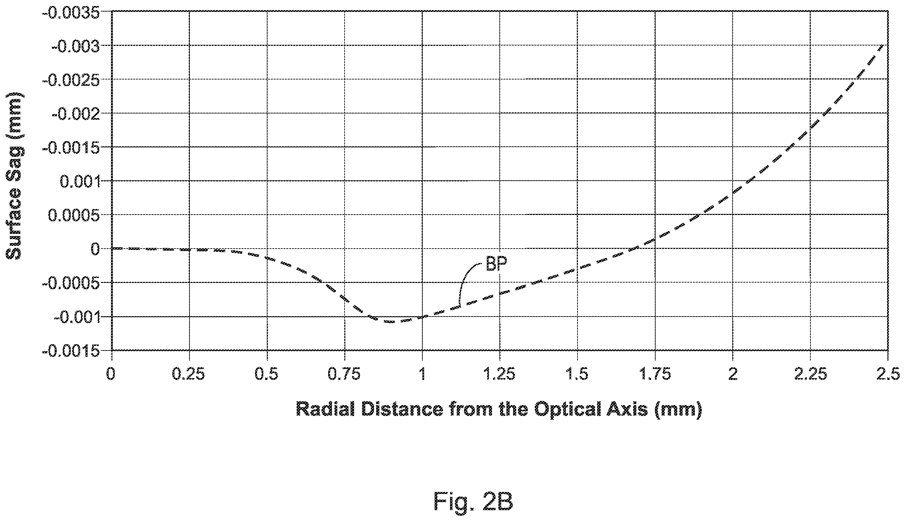

FIG. 2B is graphical illustration of a sagittal surface profile of the EDOF features of a surface of an EDOF lens as shown in FIG. 2A with the sagittal surface profile contribution of the base lens subtracted out. In particular, the conventional component of the surface (i.e., as determined by the conic term of Equation 1(a), below, is omitted; and the aspects of the surface determined by fourth order term $\alpha(r)r^4$ are illustrated).

FIG. 3 is a graphical illustration of a power profile 300 of a 20-diopter lens including the lens surface 112 (shown in FIG. 1A) illustrated in FIG. 2A, in which the opposing surface has a uniform power or a substantially uniform power (i.e., within about +/- 10%). In the illustrated example power profile, a first region $R_1$ corresponds to the first region $R_1$ in FIG. 2 (i.e., where the curvature of the lens is increasing). In first region $R_1$, the power profile is increasing as a function of increasing radial distance across the entire first region, such that a lens power greater than the base power $\Phi_{base}$ is achieved. First region $R_1$ is disposed from optical axis OA to outer edge OE of the first region. Although surface 100 is illustrated as having increasing power across the entirety of first region $R_1$, the first region may have one or more regions where the power is uniform as a function of radial distance. Accordingly, first region $R_1$ may have a power profile that is non-decreasing as a function of radial distance.

Although, in the illustrated embodiment, the lens has a power corresponding to base power $\Phi_{base}$ at the optical axis, such a configuration is not necessary. The purpose of first region $R_1$ is to spread light along the optical axis and, when combined with light from the remainder of the lens, a focal spread along a depth of focus extending from distance vision toward intermediate vision is provided. The increase in power $\Delta D_1$ from the optical axis to outer edge OE is typically in the range 0.5 diopters to 5 diopters, where 0.5 diopters provides a relatively small depth of focus and 5 diopters provides a relatively large depth of focus.

The increase in the power that occurs within the first region is selected to be great enough to provide sufficient spread of light along the optical axis (i.e., to achieve a DOF of at least about 1 diopters to about 1.5 diopters) but not so great as to unreasonably increase the likelihood of dysphotopsia. A performance goal of lenses according to some aspects of the present invention is a single peak in perceived resolution (i.e., the lens is monofocal) with a depth of focus around the peak toward the myopic direction.

It is to be appreciated that variation in power $\Delta D_1$ in first region $R_1$ is not equivalent to add power of the lens. Since each power (including the peak power) may occur only over an infinitesimally small radial extent, the add power perceived by a wearer is less than variation in power $\Delta D_1$.

As shown in FIG. 3, the power profile has a second region $R_2$ (corresponding to the second region $R_2$ in FIG. 1) that extends radially outward from outer edge OE. In second region R2, the power profile decreases from outer edge OE to a minimum lens power MP that is below the base power $\Phi_{base}$, and then increases to achieve the base power $\Phi_{base}$ at location BP. As stated above, second region R2 has substantially the base power present over at least 30% of the radial distance corresponding to the pupil of the user's eye for photopic vision conditions.

The decrease in power (in the second region) to a power less than the base power $\Phi_{base}$ may occur as a step in power (i.e., an abrupt change in curvature) at outer edge OE. It is to be appreciated that a step in power will result in the location of the outer edge and the location of minimum lens power being radially coincident or substantially radially coincident (i.e., as determined by manufacturing and measurement tolerances). Alternatively, the decrease in power may be achieved using a smoothly varying function. Typically, the decrease in power between the outer edge and minimum lens power MP happens relatively rapidly as a function of radial position (e.g., over a distance of less than 0.4 mm or less than 0.3 mm); for example, if the decrease does not occur as a step in power, a continuous function having a curvature that varies rapidly (and continuously) as function of radial position may be used. For example, a high-order polynomial (e.g., 24th order or greater) or another continuous function as described below, may be used.

In the illustrated embodiment, a power profile is achieved with the posterior surface having EDOF features (as described above) and the anterior surface having a substantially uniform power profile across the entire surface; however, either the anterior surface or the posterior surface can have EDOF features. In some embodiments, a curvature of at least one of the anterior surface and the posterior surface varies to achieve a power profile as described above; however, in some embodiments, an index of refraction of IOL 100 varies to achieve a power profile (i.e. ,the lens is a GRIN lens). The variation in index may provide the entire power of the lens (i.e., surfaces of the lens are planar); alternatively, one or both of the surfaces of a lens may be curved to provide some power.

One example of a set of equations suitable for generating a sagittal surface profile of a lens capable of providing an extended depth of focus as set forth above with reference to FIG. 2A and suitable for providing EDOF features limited to an inner portion of a photopic pupil as discussed above is given by Equations 1(a) and 1(b). These equations are also suitable for providing substantially the base power over at least 30% of the radial distance corresponding to the pupil of the user's eye for photopic vision conditions.

$$Z(r) = r^2\left\{R + \sqrt{R^2 - (1+c)r^2}\right\}^{-1} + \propto (r) * r^4 \quad - \quad \text{Equation } 1(a)$$

$$\propto (r) = \begin{cases} r \leq r_{lim}; & \propto_1 + 2(\propto_2 - \propto_1)\{(1 + e^{-Ar})^{-1} - 0.5\} \\ r > r_{lim}; & \propto_1 \end{cases} \quad - \quad \text{Equation } 1(b)$$

where r is the radial distance from the lens optical axis,
R is the surface radius of curvature,
c is the surface conic constant,
$\alpha_1$, $\alpha_2$ and A are constants defining variation of the fourth order coefficient as a function of r,
$r_{lim}$ is the location of the outer edge of the first region and defines a radial transition location for the fourth order coefficient ($\alpha (r)$).

According to Equations 1(a) and 1(b), a surface is characterized by a SAG defining a curvature that increases from the optical axis OA to the outer edge of the first zone ($r_{lim}$) using a fourth-order SAG term that increases from a value $\alpha_1$ (typically corresponding to base power) at the optical axis to a value $\alpha_2$ at the outer edge of the first zone. For values of r greater than $r_{lim}$, the fourth order coefficient term is equal to a fixed value $\alpha_1$ and thus achieves base power for outer radial locations on the lens (i.e., over at least 30% of the radial distance corresponding to the pupil of the user's eye for photopic vision conditions). Although only a single term $\alpha(r)*r^4$ is shown, additional higher order terms (e.g., $\alpha_6(r)*r^6$ and/or $\alpha_8(r)*r^8$, etc.) may also be included.

Below is another set of equations 1(c) - 1(d) capable of providing an extended depth of focus as set forth above and EDOF features limited to an inner portion of a photopic pupil.

$$Z(r) = r^2 \left\{ R + \sqrt{R^2 - (1+c)r^2} \right\}^{-1} + \propto (r) * r^4 \quad - \quad \text{Equation } 1(c)$$

$$\propto (r) = \begin{cases} r \leq r_{lim}; & \propto_1 + 2(\propto_2 - \propto_1)\{(1 + e^{-Ar})^{-1} - 0.5\} \\ r > r_{lim}; & \propto_2 + 2(\propto_1 - \propto_2)\left\{\left(1 + e^{-B(r - r_{lim})}\right)^{-1} - 0.5\right\} \end{cases} \quad \text{Equation } 1(d)$$

where, B is a constant chosen for appropriate smoothening.

According to Equations 1(c) and 1(d), a surface is characterized by a SAG defining a curvature that increases from the optical axis OA to the outer edge of the first zone ($r_{lim}$) using a fourth order SAG term having a coefficient that increases from a value $\alpha_1$ (corresponding to a base power) at the optical axis to a value $\alpha_2$ at the outer edge of the first zone. For values of r greater than $r_{lim}$, the coefficient of the fourth order SAG term begins at a value $\alpha_2$ and returns to a value $\alpha_1$ and thus achieves substantially the base power for outer radial locations on the lens (i.e., over at least 30% of the radial distance corresponding to the pupil of the user's eye for photopic vision conditions).

According to Equations 1(c) - 1(d), a surface has a radius of curvature that increases from the optical axis OA to an edge of the first zone $r_{lim}$ to achieve a curvature corresponding to a power greater than base power. For values of r greater than $r_{lim}$, the radius of curvature of the surface begins at a curvature corresponding to a power greater than base power value, then the curvature decreases to curvatures corresponding to less than base power, and then returns to curvatures corresponding to substantially the base power for outer radial position on the lens. As discussed with equation 1(a) above, although equation 1(c) is shown having only a single term $\alpha(r)*r^4$, additional higher order terms (e.g., $\alpha_6(r)*r^6$ and/or $\alpha_8(r)*r^8$) may also be included.

In some instances, the surface z(r) is further smoothened at the radial position $r_{lim}$ to eliminate a step in the SAG profile that may occur at $r_{lim}$ for surfaces defined by equations 1(c) - 1(d). Equations 1(e) - 1(g) are capable of generating a sagittal surface profile having characteristics as discussed above with reference to FIG. 2A.

$$Z(r) = r^2 \left\{ R + \sqrt{R^2 - (1+c)r^2} \right\}^{-1} + \propto (r) * r^4 + z_{shift} \quad - \quad \text{Equation } 1(e)$$

$$\propto (r) = \begin{cases} r \leq r_{lim}; & \propto_1 + 2(\propto_2 - \propto_1)\{(1 + e^{-Ar})^{-1} - 0.5\} \\ r > r_{lim}; & \propto_2 + 2(\propto_1 - \propto_2)\left\{\left(1 + e^{-B(r - r_{lim})}\right)^{-1} - 0.5\right\} \end{cases} \quad \text{Equation } 1(f)$$

$$z_{shift} = \begin{cases} & r \leq r_{lim}; 0 \\ r > r_{lim}; & (\propto_{lim} (r_{lim})^4 - \propto_1 (r_{lim})^4)\left(2\left(\left(1 + e^{-B(r - r_{lim})}\right)^{-1} - 0.5\right)\right) \end{cases} -$$

$$\text{Equation } \quad 1(g)$$

As discussed with equation 1(a) above, although equations 1(c) and 1(e) are shown having only a single term $\alpha(r)*r^4$, additional higher order terms (e.g., $\alpha_6(r)*r^6$ and/or $\alpha_8(r)*r^8$ etc.) may also be included.

Like Equations 1(c) and 1(d), Equations 1(e) - 1(f) define a surface characterized by a SAG defining a curvatures that increase from the optical axis OA to the outer edge of the first zone ($r_{lim}$) using a fourth order SAG coefficient term that increases from a value $\alpha_1$ (corresponding to base power) at the optical axis to a value $\alpha_2$ at the outer edge of the first zone. For values of r greater than $r_{lim}$, the fourth order SAG coefficient term begins at a value $\alpha_2$ and returns to a value $\alpha_1$ and thus provides curvatures corresponding substantially to the base power for outer radial locations on the lens (i.e., over at least 30% of the radial distance corresponding to the pupil of the user's eye for photopic vision conditions). Additionally, Equation 1(g) provides an offset in the sag ($z_{shift}$) to eliminate any step at $r_{lim}$.

It is to be understood that a discontinuous change in curvature and a step in sagittal height as described above do not effect a lens's ability to provide an extended depth of focus; however, during examination of a lens prior to or after surgical implantation, a step may be readily apparent when illuminated with a light source. Accordingly, in some embodiments, it is advantageous that a surface is smoothened using Equations 1(c) - 1(d) or Equations 1(e) - 1(g).

The sagittal surface profile of lens 100 shown in FIG. 2A was generated from Equations 1(e) - 1(g) using the following values:

R = 21.722 mm

c = -1.2257

$$\alpha_1 = -1.0 \times 10^{-4}$$

$$\alpha_2 = 24.0 \times 10^{-4},$$

and
$r_{lim}$ = 0.75 mm
A = - 9.0
B = - 9.0

Although in the above equations, Z(r), only a single term $\alpha(r)*r^4$ is shown, additional higher order terms (e.g., $\alpha_6(r)$ $*r^6$ and/or $\alpha_8(r)*r^8$) may also be included.

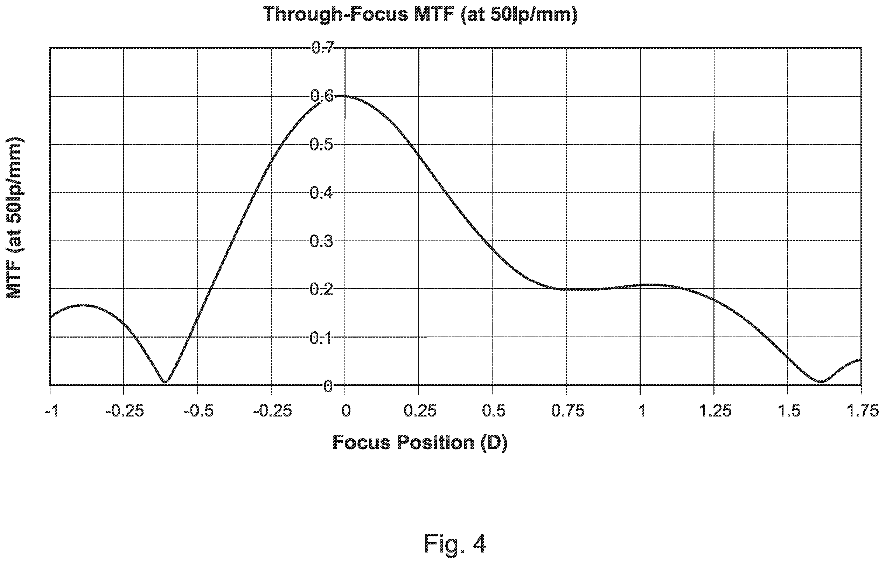

FIG. 3 is a power profile, and FIG. 4 is a graphical illustration of a through-focus MTF, both, calculated using the above values to produce a posterior surface and having an anterior surface that is spherical and has a radius of curvature of 21.722 mm to form a 20-diopter lens. In particular, for the present example, and for all MTFs specified herein the MTF is calculated using the ISO 1 model eye, and the MTF is calculated for a 50 lp/mm image at the retina, for 546 nm light, and a 3 mm pupil. For all calculations made herein, lenses are assumed to be immersed in aqueous humor (refractive index of 1.336 at 546 nm).

As will be understood in the art, a power profile can be calculated, for example, using surface curvatures or can be measured using a suitable metrological instrument such as a wavefront measurement system.

EDOF features are present at locations radially inward of point BP (i.e., the location where base power is attained after minimum power MP). For example, the EDOF features may be located within a radius of 1.4 mm or 1.2 mm or 0.7 mm about the optical axis. It will be appreciated that the radial extent of the EDOF features is determined by constants $\alpha_1$, $\alpha_2$, A and $r_{lim}$ in the equations above.

In FIG. 4, the 0 diopter (D) location on the horizontal axis corresponds to distance vision and positive dioptric values correspond to amounts of myopic defocus. In some embodiments, an EDOF IOL is designed to have a best focus (i.e., an absolute MTF maximum) at far vision and a depth of focus extending toward near vision. It is apparent that acuity at far vision is relatively high MTF (0.5 or greater) and a depth of focus (i.e., locations continuous with the 0 diopter location and having an MTF greater than or equal to 0.15) of about 1.25 diopters is achieved. The lens has only a single peak in the MTF plot and is therefore monofocal.

As will be understood in the art, MTFs can be calculated in a straightforward numerical manner, using a raytracing program such as Oslo® from Sinclair Optics of Pittsford NY or Zemax from Zemax, LLC of Kirkland, WA or by another existing simulation tool, or by self-written code, all of which provide equivalent results.

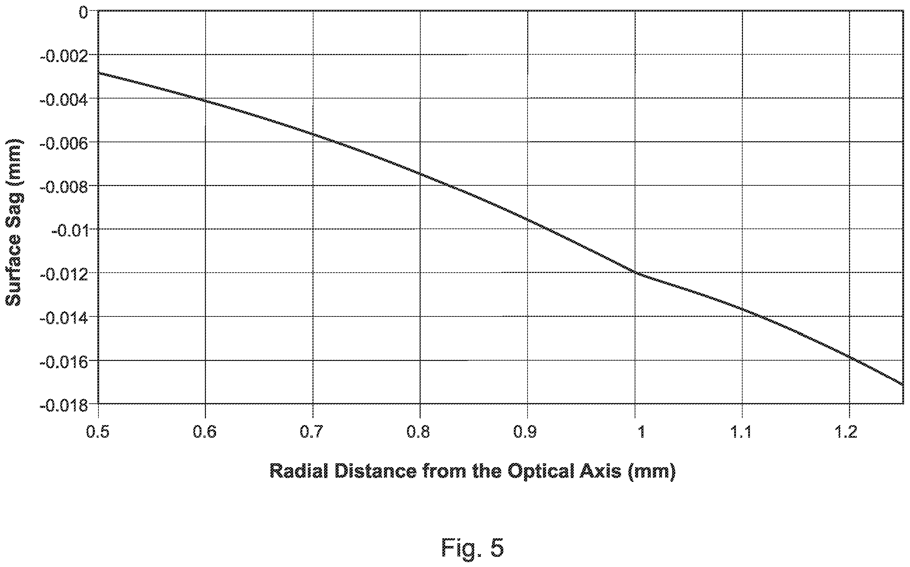

FIG. 5 illustrates a portion of a second example of a sagittal surface profile of a surface of an EDOF lens according to aspects of the invention. The illustrated portion of the sagittal surface profile shows a portion of the profile proximate $r_{lim}$. The surface corresponds to a relatively low power lens (e.g., 6 diopters). The surface was generated using Equations 1(a) - 1(b). The illustrated portion includes the discontinuous change in curvature and includes a step in the sagittal surface profile at $r_{lim}$ (at about 1.0 mm from the optical axis). A surface of a relatively low-power lens was chosen because the variation in sagittal height proximate $r_{lim}$ caused by changes in curvature specified by Equations 1(a) and 1(b) are most apparent. While the effects of the change in curvature is readily visible in FIG. 5, the step is relatively small as compared to the overall height variation of z(r) across the illustrated portion, and thus is not readily visible in FIG. 5.

The surface sagittal profile of the surface of FIG. 5 was generated using the following values:

R = 45 mm
C = -1.2257

$$\alpha_1 = -1.0 \times 10^{-4}$$

$$\alpha_2 = 7.0 \times 10^{-4}$$

A = -4, and
B = - 20

The above sag equations (1(a)-1(b), 1(c) - 1(d) and 1(e) - 1(g)) illustrate that an aspheric SAG profile having a conic term and a fourth order term with an exponential coefficient (i.e., the SAG varies exponentially) is one technique to achieve a relatively rapid increase in a power profile to allow EDOF features to be maintained in the inner portion of the radius of the lens as set forth above, however, other mathematical equations can be used to achieve the desired rapid increase in power profile (and possibly a rapid decrease in power profile). For example, a surface can be described by a spline, a Bezier curve, or a SAG profile as shown in Equation 1(a) in which a sixth order or higher aspheric term is included (which may be in addition to a fourth order term or without a fourth order term; and terms lower than fourth order are not present), a high-order polynomial (e.g., 24th order or greater), or an empirically-derived SAG function which provides for variations in surface curvature as a function of r.

One example of another type of equations suitable for generating a SAG profile of a lens capable of limiting the EDOF features to an inner portion of the photopic pupil and providing substantially the base power profile over at least 30% of the radial distance corresponding to the pupil of the user's eye for photopic vision conditions is given by Equation 2. It will be appreciated that Equation 2 is an example of an equation suitable for specifying SAG using a high-order polynomial equations (e.g., 24th or greater order ($m \geq 12$)).

$$z(r) = r^2 \left\{ R + \sqrt{R^2 - (1+c)r^2} \right\}^{-1} + \sum_{n=1}^{m} C_{2n} r^{2n} \quad - \quad \text{Equation 2}$$

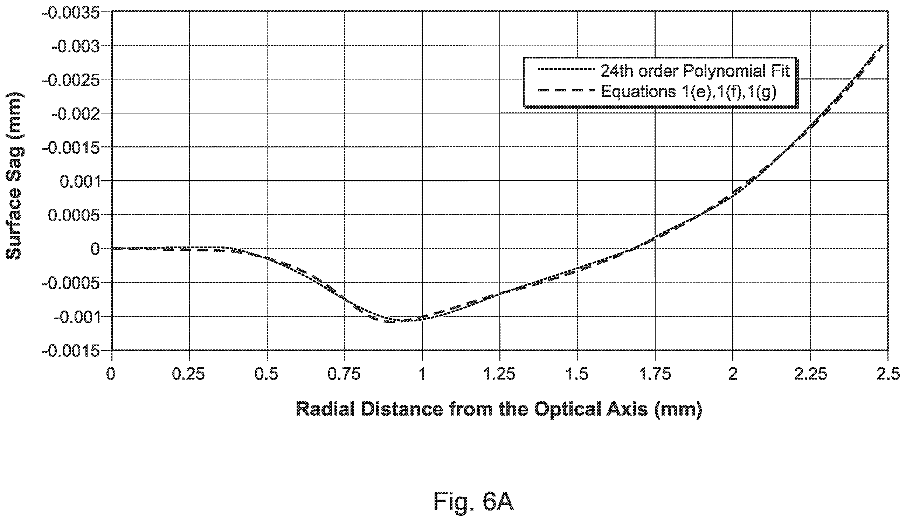

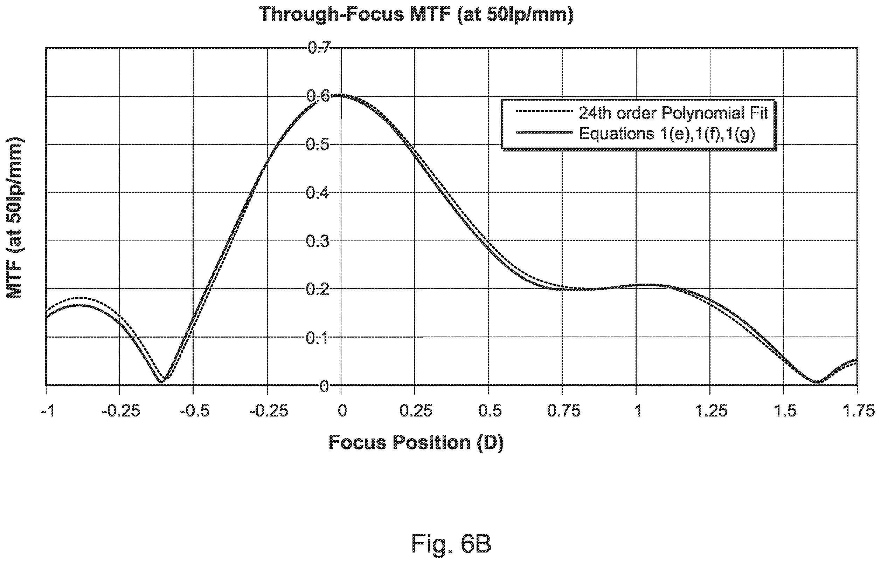

FIG. 6A is a sagittal surface profile of an example of EDOF features of a surface of an EDOF lens (i.e., the conic term is omitted) in which the EDOF features are specified using a polynomial expression as shown in Equation 2. In particular, FIG. 6 illustrates the SAG of an EDOF features where

$$C_2 = 2.2312345 \times 10^{-04}$$

$$C_4 = -8.6023283 \times 10^{-04}$$

$$C_6 = -1.9063097 \times 10^{-02}$$

$$C_8 = 5.1027672 \times 10^{-02}$$

$$C_{10} = -5.8342430 \times 10^{-02}$$

$$C_{12} = 3.8059413 \times 10^{-02}$$

$$C_{14} = -1.5613206 \times 10^{-02}$$

$$C_{16} = 4.1760810 \times 10^{-03}$$

$$C_{18} = -7.2856545 \times 10^{-04}$$

$$C_{20} = 8.0014765 \times 10^{-05}$$

$$C_{22} = -5.0251952 \times 10^{-06}$$

$$C_{24} = 1.3766880 \times 10^{-07}$$

FIG. 6A also shows the sagittal surface profile of FIG. 2A for comparison. It will be appreciated that coefficients of a 24th order polynomial expression can be selected, for example, to provide a SAG that closely approximates the SAG generated using Equations 1(e) - 1(g) from the optical axis to the edge of the lens. Although a surface generated using Equations 1(e) - 1(g) was approximated for illustration, a surface generated using Equations 1(a) - 1(b) or 1(c) - 1(d) can be similarly approximated using a high-order polynomial expression.

For example, the EDOF features can be used to form a surface where R = 21.722 mm and c = -1.2257 are used in the conic term of Equation 2 to form a posterior surface. Such a posterior surface may be used in combination with a spherical anterior surface having a radius of curvature of 21.722 mm to form a 20-diopter lens.

FIG. 6B is a graphical illustration showing that a through-focus MTF for the 20-diopter lens using the EDOF features defined by a $24^{th}$ order polynomial set forth with reference to FIG. 6A is very similar to the through-focus MTF of the surface generated using Equations 1(e) - 1(g). Accordingly, given the similar SAG and MTF of the lens having a SAG specified using a polynomial and the lens specified using Equations 1(e) - 1(g), it can be expected that a power profile of the lens specified using a polynomial will provide a similar power profile to that shown in FIG. 2B.

For example, the decrease in power extending outward from the outer edge of the first zone to a value less than the base power happens relatively rapidly as a function of radial position (e.g., over a distance of less than 0.4 mm or less than 0.3 mm).

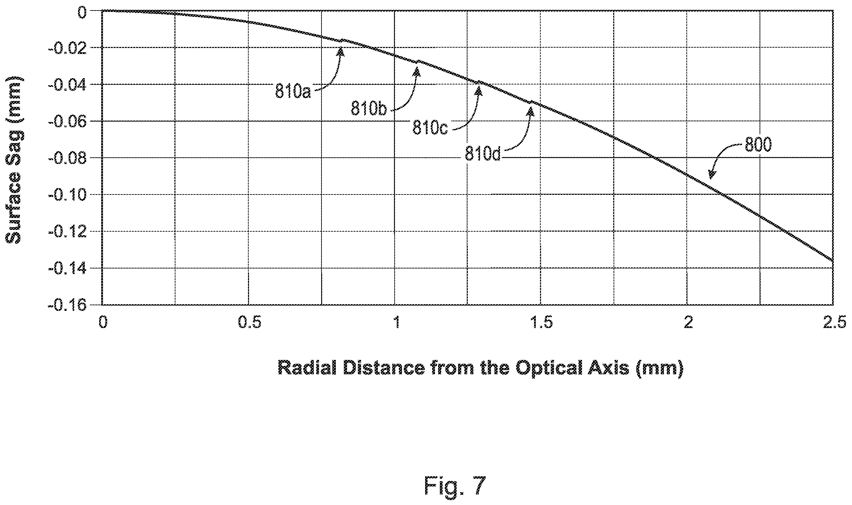

In some embodiments, a diffractive profile is added to a lens surface configured to provide EDOF capabilities as described above to form a refractive-diffractive lens. For example, the diffractive profile may be superimposed on a surface containing a refractive EDOF design as set forth above (e.g., the diffractive profile provides add power to a surface specified using equations 1(a) - 1(b) or equations 1(c) - 1(d) or equations 1(e) - 1(g)). FIG. 7 illustrates an example of a sagittal surface profile of a surface 800 of a refractive-diffractive EDOF lens according to aspects of the present invention. In the illustrated embodiment, the diffractive profile comprises four zones defined by zone edges 810a, 810b, 810c and 810D. The illustrated embodiment of a diffractive profile is of a bifocal configuration (having a zeroth order peak corresponding the base power of the lens and a second peak corresponding to an add power provided by the diffractive profile), however a diffractive profile may be of a multifocal configuration (having one or more additional peaks with corresponding add powers). While a diffractive profile may be of a multifocal design, the extended depth of focus which is provided by the refractive EDOF features will typically increase MTF values in a region of the depth of focus between a first peak (i.e., corresponding to far focus) generated by the diffractive profile and a second peak (i.e., a neighboring peak (the first peak in the myopic direction)) generated by the diffractive profile. In some embodiments, the increase in MTF only occurs between the first peak and second peak; however, in some embodiments, the increase in MTF occurs predominantly in the region between the first and second peaks, but extends beyond this region. Typically, it is desirable that the entire increase in MTF occurs in the myopic direction from the first peak rather than in the hyperopic direction.

The example refractive-diffractive sagittal surface profile illustrated in FIG. 7 is specified by equations 1(e) - 1(g) where the following values were used:

R = 21.722 mm
c = -1.2257
$\alpha_1$ = -0.000197
$\alpha_2$ = 0.0005
A=-9,
B = -9, and

the diffractive profile is specified as follows: diffractive add power of 2.2 D, design wavelength of 546 nm, diffractive step height of 1.218 $\mu$m, and a central refractive zone of 0.4 mm radius. The diffractive profile, by itself, is of a conventional design for use in an IOL.

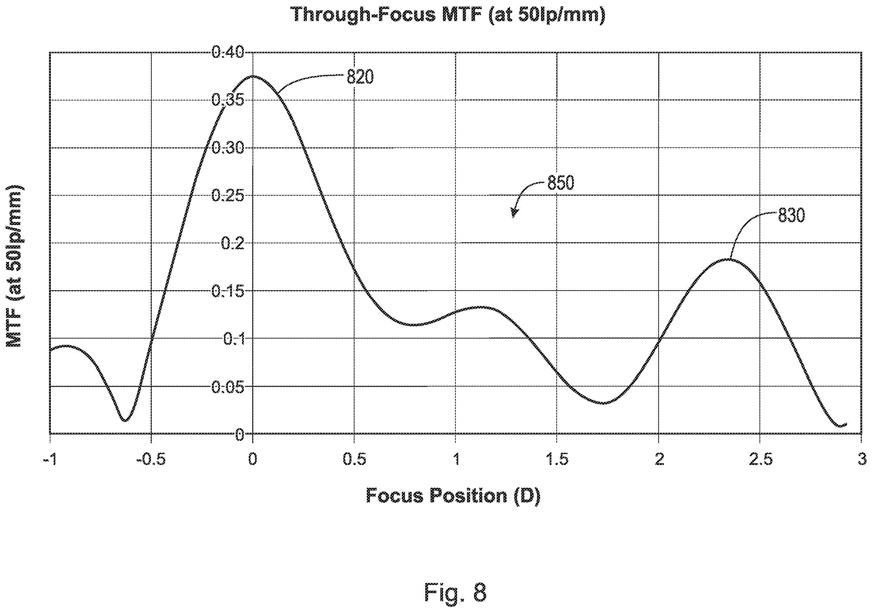

FIG. 8 is a graphical illustration of a through-focus MTF for a lens including the example surface as shown in FIG. 7. The lens used to generate the MTF is a 20 diopter lens in which the surface opposing surface 800 is a spherical surface having a radius of curvature of 18 mm. The bifocal diffractive profile directs light to peaks 820 and 830 and the refractive EDOF features direct light into region 850.

It is apparent that acuity at far vision (0 diopters) is relatively high (i.e., MTF in excess of 0.35) and a depth of focus (i.e., locations continuous with the 0 diopter location and having an MTF greater than 0.1) of about 1.3 diopters is achieved. The lens has two peaks in the MTF plot (using values greater than 0.1) and is therefore bifocal. It is to be appreciated that, while the depth of focus does not extend the full distance between the peaks 820 and 830, eye aberrations of selected wearers can be expected to cause the spread of light energy from the peaks to portions of the depth of focus between the peaks thus allowing continuous vision for a wearer, over about a 3 diopter depth of focus. To achieve the desired perceived depth of focus, in the some embodiments, the first peak in the MTF (corresponding to far vision) and the neighboring peak in MTF (in the myopic direction) are separated by about 2.5 diopters, and a depth of focus continuous with the first peak is at least about 1.25 diopters.

Various embodiments have been depicted and described in detail herein, it will be apparent to those skilled in the relevant art that various modifications, additions, substitutions, and the like can be made without departing from the scope of the invention as defined in the claims which follow.

**Claims**

1.  An intraocular lens providing an extended depth of focus, the lens having an optical axis and a base power for achieving distance vision, the lens comprising:
    an optic having a surface with a sagittal surface profile defined in-part by EDOF features having a first region of increasing power as a function of radial position and_that is non-decreasing in curvature as a function of increasing radial position from the optical axis to an outer edge of the first region to achieve powers greater than the base power at the outer edge, the increasing power sufficient to achieve a depth of focus extending from distance vision toward intermediate vision of 1 diopters to 1.5 diopters, and the sagittal surface profile having a second region extending radially outward from the outer edge that is non-increasing in curvature as a function of increasing radial position from the outer edge to achieve powers less than the base power thereby defining a non-increasing portion, the curvatures in the second region then non-decreasing to achieve the base power and then maintaining substantially the base power over at least over a radial distance of at least 0.3 mm .

2.  The lens of claim 1, wherein the first region is increasing in curvature as a function of

    increasing radial position from the optical axis to the outer edge of the first region, the second region is decreasing in curvature as a function of increasing radial position from the outer edge to achieve the powers less than the base power, and then increasing in curvature as a function of radial position to achieve the base power, and/or wherein substantially the base power is maintained in the second region over a radial distance of at least 0.6 mm.

3.  The lens of claim 1, wherein the sagittal surface profile can be specified by an equation z(r),

    where

    $$z(r) \;=\; \mathrm{r}^2 \left\{ R + \sqrt{R^2 - (1+c)r^2} \right\}^{-1} + \propto_m (r) * r^m \quad,$$

    and
    where m is 4 or higher.
    r is the radial distance from the lens optical axis,
    R is the surface radius of curvature,
    c is the surface conic constant,
    $\alpha_1$, $\alpha_2$ and A are constants defining variation of the fourth order coefficient as a function of r,
    $r_{lim}$ is the location of the outer edge of the first region and defines a radial transition location for the fourth order coefficient ($\alpha$ (r)), and preferably

    wherein m = 4, and preferably
    where $\alpha_m$(r) varies exponentially as a function of radial distance, and preferably
    wherein

    $$\propto_m (r) = \begin{cases} \mathrm{r} \leq r_{lim}; & \propto_1 + 2(\propto_2 - \propto_1)\{(1 + e^{-Ar})^{-1} - 0.5\} \\ \mathrm{r} > r_{lim}; & \text{the coefficeint } \propto_m \text{ is defined to attain} \\ & \qquad \text{a value } \propto_1 \end{cases}$$

    r is the radial distance from the lens optical axis,
    R is the surface radius of curvature,
    c is the surface conic constant,
    $\alpha_1$, $\alpha_2$ and A are constants defining variation of the fourth order coefficient as a function of r,
    $r_{lim}$ is the location of the outer edge of the first region and defines a radial transition location for the fourth order coefficient ($\alpha$ (r)).

4.  The lens of claim 3, wherein substantially the base power is maintained in the second region over a radial distance of at least 0.6 mm, and/or

wherein for r > $r_{lim}$
$\propto$ (r) = $\propto_1$ whereby $\alpha(r)$ attains value $\alpha_1$ at $r_{lim}$, and/or
wherein for r > $r_{lim}$

$$\propto (r) = \propto_2 + 2(\propto_1 - \propto_2)\left\{\left(1 + e^{-B(r-r_{lim})}\right)^{-1} - 0.5\right\}$$

where, B is a constant,
whereby $\alpha(r)$ attains value $\alpha_1$ gradually for values of r greater than $r_{lim}$, and/or wherein z(r) further comprises a term $z_{shift}$
where

$$z_{shift} = \begin{cases} r \leq r_{lim}; 0 \\ \text{r} > r_{lim}; \ (\propto_{lim} (r_{lim})^4 - \propto_1 (r_{lim})^4)\left(2\left(\left(1 + e^{-B(r-r_{lim})}\right)^{-1} - 0.5\right)\right) \end{cases}$$

5. The lens of claim 1, wherein the sagittal surface profile can be specified by an equation z(r), where

$$z(r) = r^2\left\{R + \sqrt{R^2 - (1+c)r^2}\right\}^{-1} + \text{EDOF features sagittal profile term}$$

and, wherein the EDOF features sagittal profile term can be specified using a polynomial expression, and preferably

wherein the polynomial expression approximates an exponential variation, and/or
wherein the polynomial expression is of a form $\sum_{n=1}^{m} C_{2n} r^{2n}$ , and/or
wherein the polynomial expression is of 24$^{th}$ order or greater, whereby m $\geq$ 12.

6. The lens of claim 1, wherein the surface is an anterior surface or a posterior surface, and wherein the non-increasing curvature portion is **characterized by** a discontinuity located at the outer edge, such that the outer edge and the location of minimum lens power are substantially radially coincident.

7. The lens of claim 1, wherein the surface is an anterior surface or a posterior surface, and

wherein the non-increasing curvature portion varies smoothly as a function of radius such that the distance from the outer edge of the first region to a radial location of a minimum lens power is less than 0.4 mm, and/or
wherein the lens is monofocal, and/or
wherein the sagittal surface profile is further defined by a diffractive profile superimposed on the surface.

8. The lens of claim 7, wherein the diffractive profile is configured to produce a depth of focus having a first peak in MTF and a second peak in MTF, and wherein the EDOF features increases the MTF between the first peak and the second peak, wherein the MTF is specified using the ISO 1 model eye, and wherein the MTF is specified for a spatial frequency of 50 lp/mm at the retina, at 546 nm light, for a 3 mm diameter pupil, and when the lens is immersed in aqueous humor having a refractive index of 1.336 at 546 nm, and preferably

wherein the EDOF features increases the MTF only between the first peak and the second peak, and/or
wherein the diffractive profile is a bifocal diffractive profile, and/or
wherein the maximum MTF value is equal to or greater than 0.35.

9. The lens of claim 8, wherein the first peak in the MTF and the second peak are separated by about 2.5 diopters, and the EDOF features provides a depth of focus continuous with the first peak that maintains an MTF of 0.15 or greater for at least about 1.25 diopters, in the myopic direction from the first peak.

10. An intraocular lens providing an extended depth of focus, the lens having an optical axis and a base power for achieving distance vision, the lens comprising:

an optic **characterized by** a power profile that, in a first region of increasing power as a function of radial position, is non-decreasing as a function of increasing radial position from the optical axis to an outer edge of the first region to achieve a lens power greater than the base power at the outer edge, the increasing power sufficient to achieve a depth of field extending from distance vision toward intermediate vision of 1 diopters to 1.5 diopters and, in a second region extending radially outward from the outer edge the power profile is non-increasing as a function of increasing radial position from the outer edge to achieve a minimum lens power that is less than the base power thereby defining a non-increasing portion, and then non-decreasing as a function of increasing radial position to achieve the base power, the second region then maintaining substantially the base power over at least over a radial distance of at least 0.3 mm .

11. The lens of claim 10, wherein the first region is increasing in power as a function of increasing radial position from the optical axis to the outer edge of the first region, the second region is decreasing in power as a function of increasing radial position from the outer edge to achieve the minimum lens power, and then increasing in curvature as a function of radial position to achieve the base power, and/or

    wherein the increase in power across the first region is in the range of 0.5 to 5.0 diopters, and preferably wherein the lens achieves a perceived depth of focus in the range of 1.0 to 1.5 diopters.

12. The lens of claim 10, wherein the lens is monofocal, and/or

    wherein there is a step in power at the outer edge, and/or
    wherein the power between the outer edge and the minimum lens power is smoothly varying and the decrease in power between the outer edge and the minimum lens power occurs over a radial distance of less than 0.4 mm.

13. The lens of claim 10, wherein the lens comprises an anterior surface and a posterior surface,

    wherein the power profile is achieved by a change in surface curvature in at least one of the anterior surface and the posterior surface, and wherein the anterior surface or the posterior surface has a step in power at the outer edge of the first region, or
    wherein the lens comprises an anterior surface and a posterior surface, wherein the power profile is achieved by a change in surface curvature in at least one of the anterior surface and the posterior surface, and wherein neither the anterior surface nor the posterior surface has a step in power at the outer edge of the first region, or
    wherein the lens comprises an anterior surface and a posterior surface, wherein the power profile is achieved by a change in surface curvature in at least one of the anterior surface and the posterior surface, and wherein the non-increasing portion of the second region is **characterized by** a power as a function of radius that is smoothly varying such that the distance from the outer edge of the first region to a radial location of the minimum lens power is less than 0.4 mm.

14. The lens of claim 10, wherein the lens is a gradient index lens having an index of refraction that varies radially to provide the power profile and/or

    wherein substantially the base power is maintained in the second region over a radial distance of at least 0.6 mm, and/or
    wherein the lens further comprises a surface having a diffractive profile superimposed thereon.

15. The lens of claim 14, wherein the diffractive profile is configured to produce a depth of focus having a first peak in MTF and a second peak in MTF, and wherein the power profile increases the MTF between the first peak and the second peak, wherein the MTF is specified using the ISO 1 model eye, and wherein the MTF is specified for a spatial frequency of 50 lp/mm at the retina, at 546 nm light, for a 3 mm diameter pupil, when the lens is immersed in aqueous humor having a refractive index of 1.336 at 546 nm, and preferably

    wherein the power profile increases the MTF only between the first peak and the second peak, and/or
    wherein the diffractive profile is a bifocal diffractive profile, and/or
    wherein the maximum MTF value is equal to or greater than 0.35, and/or
    wherein the first peak in the MTF and the second peak are separated by about 2.5 diopters, and the power profile provides a depth of focus continuous with the first peak that maintains an MTF of 0.15 or greater for at least about 1.25 diopters, in the myopic direction from the first peak.

**Patentansprüche**

1. Intraokularlinse mit erweiterter Schärfentiefe, wobei die Linse eine optische Achse und eine Basisbrechkraft zum Erreichen der Fernsicht aufweist, wobei die Linse aufweist: eine Optik mit einer Oberfläche mit einem sagittalen Oberflächenprofil, das teilweise durch EDOF-Merkmale definiert ist, das einen ersten Bereich zunehmender Brechkraft als Funktion der radialen Position aufweist und dessen Krümmung als Funktion einer zunehmenden radialen Position von der optischen Achse zu einer Außenkante des ersten Bereichs nicht abnimmt, um Brechkräfte zu erzielen, die größer sind als die Basisbrechkraft an der Außenkante, wobei die zunehmende Brechkraft ausreicht, um eine Tiefenschärfe zu erreichen, die sich von Fernsicht bis Zwischensichte von 1 Dioptrie bis 1,5 Dioptrien erstreckt, und wobei das sagittale Oberflächenprofil einen zweiten Bereich aufweist, der sich radial nach außen vom äußeren Rand erstreckt und dessen Krümmung als Funktion zunehmender radialer Position vom äußeren Rand nicht zunimmt, um Brechkräfte zu erzielen, die kleiner sind als die Basisbrechkraft, wodurch ein nicht zunehmender Abschnitt definiert wird, wobei die Krümmungen im zweiten Bereich dann nicht abnehmen, um die Basisbrechkraft zu erzielen, und dann die Basisbrechkraft über mindestens einen radialen Abstand von mindestens 0,3 mm im Wesentlichen aufrechterhalten wird.

2. Linse nach Anspruch 1, wobei der erste Bereich eine zunehmende Krümmung als Funktion zunehmender radialer Position von der optischen Achse zum äußeren Rand des ersten Bereichs aufweist, der zweite Bereich eine abnehmende Krümmung als Funktion zunehmender radialer Position vom äußeren Rand aufweist, um die Brechkräfte zu erzielen, die kleiner sind als die Basisbrechkraft, und dann eine zunehmende Krümmung als Funktion der radialen Position aufweist, um die Basisbrechkraft zu erzielen, und/oder wobei im Wesentlichen die Basisbrechkraft im zweiten Bereich über einen radialen Abstand von mindestens 0,6 mm aufrechterhalten wird.

3. Linse nach Anspruch 1, wobei das sagittale Oberflächenprofil durch eine Gleichung z(r) spezifiziert werden kann,

   wobei

$$z(r) = r^2 \left\{ R + \sqrt{R^2 - (1+c)r^2} \right\}^{-1} + \alpha_m(r) * r^m,$$

   und
   wobei m 4 oder größer ist,
   r der radiale Abstand von der optischen Achse der Linse ist,
   R der Oberflächenkrümmungsradius ist,
   c die konische Konstante der Oberfläche ist,
   $\alpha_1$, $\alpha_2$ und A Konstanten sind, die die Variation des Koeffizienten vierter Ordnung als Funktion von r definieren,
   $r_{lim}$ die Position des äußeren Randes des ersten Bereichs ist und eine radiale Übergangsposition für den Koeffizienten vierter Ordnung ($\alpha$(r)) definiert, und vorzugsweise
   wobei m = 4 und vorzugsweise
   wobei $\alpha_m$(r) exponentiell als Funktion des radialen Abstands variiert, und vorzugsweise
   wobei

$$\alpha_m(r) = \begin{cases} r \leq r_{lim}; & \alpha_1 + 2(\alpha_2 - \alpha_1)\{(1 + e^{-Ar})^{-1} - 0.5\} \\ r > r_{lim}; & \text{der Koeffizient } \alpha_m \text{definiert ist, um einen Wert } \alpha_1 \\ & \text{zu erreichen} \end{cases}$$

   r der radiale Abstand von der optischen Achse der Linse ist,
   R der Oberflächenkrümmungsradius ist,
   c die konische Oberflächenkonstante ist,
   $\alpha_1$, $\alpha_2$ und A Konstanten sind, die die Variation des Koeffizienten vierter Ordnung als Funktion von r definieren,
   $r_{lim}$ die Position des äußeren Randes des ersten Bereichs ist und eine radiale Übergangsposition für den Koeffizienten vierter Ordnung ($\alpha$(r)) definiert.

4. Linse nach Anspruch 3, wobei im Wesentlichen die Basisbrechkraft im zweiten Bereich über einen radialen Abstand von mindestens 0,6 mm beibehalten wird und/oder wobei für $r > r_{lim}$
   $\alpha$(r) = $\alpha_1$ wobei $\alpha$(r) den Wert $\alpha_1$ bei $r_{lim}$ erreicht, und/oder

wobei für $r > r_{lim}$

$$\alpha(r) = \alpha_2 + 2(\alpha_1 - \alpha_2)\left\{\left(1 + e^{-B(r-r_{lim})}\right)^{-1} - 0.5\right\}$$

wobei B eine Konstante ist,
wobei $\alpha(r)$ allmählich den Wert $\alpha_1$ für Werte von r größer als $r_{lim}$ annimmt, und/oder

wobei z(r) ferner einen Term $z_{shift}$ aufweist, wobei

$$z_{shift} = \begin{cases} r \le r_{lim}; & 0 \\ r > r_{lim}; & (\alpha_{lim}(r_{lim})^4 - \alpha_1(r_{lim})^4)\left(2\left(\left(1 + e^{-B(r-r_{lim})}\right)^{-1} - 0.5\right)\right) \end{cases}$$

5.  Linse nach Anspruch 1, wobei das sagittale Oberflächenprofil durch eine Gleichung z(r) spezifiziert werden kann, wobei

$$z(r) = r^2\left\{R + \sqrt{R^2 - (1 + c)r^2}\right\}^{-1} + \text{sagittaler Profilterm der EDOF-Merkmale}$$

und wobei der sagittale Profilterm der EDOF-Merkmale unter Verwendung eines Polynomausdrucks spezifiziert werden kann, vorzugsweise

   wobei der Polynomausdruck eine exponentielle Variation approximiert und/oder

   wobei der Polynomausdruck die Form $\sum_{n=1}^{m} C_{2n} r^{2n}$ hat, und/oder

   wobei der Polynomausdruck 24. Ordnung oder höher ist, wobei m ≥ 12.

6.  Linse nach Anspruch 1, wobei die Oberfläche eine anteriore Oberfläche oder eine posteriore Oberfläche ist und wobei der Abschnitt nicht zunehmender Krümmung durch eine Diskontinuität gekennzeichnet ist, die sich am äußeren Rand befindet, so dass der äußere Rand und der Ort der minimalen Linsenbrechkraft im Wesentlichen radial zusammenfallen.

7.  Linse nach Anspruch 1, wobei die Oberfläche eine anteriore Oberfläche oder eine posteriore Oberfläche ist und wobei der Abschnitt nicht zunehmender Krümmung als Funktion des Radius gleichmäßig variiert, so dass der Abstand vom äußeren Rand des ersten Bereichs zu einer radialen Stelle einer minimalen Linsenbrechkraft weniger als 0,4 mm beträgt, und/oder

   wobei die Linse monofokal ist, und/oder
   wobei das sagittale Oberflächenprofil ferner durch ein der Oberfläche überlagertes diffraktives Profil definiert ist.

8.  Linse nach Anspruch 7, wobei das diffraktive Profil konfiguriert ist, eine Tiefenschärfe mit einem ersten Peak in einer MTF und einem zweiten Peak in einer MTF zu erzeugen, und wobei die EDOF-Merkmale die MTF zwischen dem ersten Peak und dem zweiten Peak erhöhen, wobei die MTF unter Verwendung des ISO 1-Modellauges spezifiziert ist, und wobei die MTF für eine Raumfrequenz von 50 lp/mm an der Netzhaut, bei 546 nm Licht, für eine Pupille mit 3 mm Durchmesser und wenn die Linse in Kammerwasser mit einem Brechungsindex von 1,336 bei 546 nm eingetaucht ist, spezifiziert ist, und vorzugsweise

   wobei die EDOF-Merkmale die MTF nur zwischen dem ersten Peak und dem zweiten Peak erhöhen, und/oder
   wobei das diffraktive Profil ein bifokales diffraktives Profil ist, und/oder wobei der maximale MTF-Wert gleich oder größer als 0,35 ist.

9.  Linse nach Anspruch 8, wobei der erste Peak in der MTF und der zweite Peak um etwa 2,5 Dioptrien voneinander getrennt sind und die EDOF-Merkmale eine mit dem ersten Peak kontinuierliche Tiefenschärfe bereitstellen, die eine MTF von 0,15 oder mehr für mindestens etwa 1,25 Dioptrien in myopischer Richtung vom ersten Peak aufrechterhält.

10. Intraokularlinse, die eine erweiterte Tiefenschärfe bietet, wobei die Linse eine optische Achse und eine Basisbrechkraft zum Erreichen der Fernsicht aufweist, wobei die Linse aufweist:

eine Optik, die durch ein Brechkraftprofil gekennzeichnet ist, das in einem ersten Bereich mit zunehmender Brechkraft als Funktion der radialen Position als Funktion der zunehmenden radialen Position von der optischen Achse zu einer äußeren Rand des ersten Bereichs nicht abnimmt, um eine Linsenbrechkraft zu erzielen, die größer ist als die Basislichtbrechkraft am äußeren Rand, wobei die zunehmende Brechkraft ausreicht, um eine Tiefenschärfe zu erreichen, die sich von einer Fernsicht zum Zwischensicht von 1 Dioptrie bis 1,5 Dioptrien erstreckt, und wobei in einem zweiten Bereich, der sich radial nach außen vom äußeren Rand erstreckt, das Brechkraftprofil als Funktion der zunehmenden radialen Position vom äußeren Rand nicht zunimmt, um eine minimale Linsenbrechkraft zu erreichen, die geringer ist als die Basisbrechkraft, wodurch ein nicht zunehmender Abschnitt definiert wird, und dann nicht abnimmt als Funktion der zunehmenden radialen Position, um die Basisbrechkraft zu erreichen, wobei der zweite Bereich dann im Wesentlichen die Basisbrechkraft über mindestens einen radialen Abstand von mindestens 0,3 mm beibehält.

11. Linse nach Anspruch 10, wobei der erste Bereich eine zunehmende Brechkraft als Funktion zunehmender radialer Position von der optischen Achse zum äußeren Rand des ersten Bereichs aufweist, der zweite Bereich eine abnehmende Brechkraft als Funktion der zunehmenden radialen Position vom äußeren Rand aufweist, um die minimale Linsenbrechkraft zu erreichen, und dann eine abnehmende Krümmung als Funktion der radialen Position aufweist, um die Basisbrechkraft zu erreichen, und/oder wobei die Zunahme der Brechkraft über den ersten Bereich im Bereich von 0,5 bis 5,0 Dioptrien liegt, und vorzugsweise

wobei die Linse eine wahrgenommene Tiefenschärfe im Bereich von 1,0 bis 1,5 Dioptrien erreicht.

12. Linse nach Anspruch 10, wobei die Linse monofokal ist und/oder

wobei es am äußeren Rand eine Stufe der Brechkraft gibt, und/oder
wobei die Brechkraft zwischen dem äußeren Rand und der minimalen Linsenbrechkraft gleichmäßig variiert und die Abnahme der Brechkraft zwischen dem äußeren Rand und der minimalen Linsenbrechkraft über einen radialen Abstand von weniger als 0,4 mm erfolgt.

13. Linse nach Anspruch 10, wobei die Linse eine anteriore Oberfläche und eine posteriore Oberfläche aufweist, wobei das Brechkraftprofil durch eine Änderung der Oberflächenkrümmung in mindestens einer der anterioren Oberfläche und der posterioren Oberfläche erreicht wird und wobei die anteriore Oberfläche oder die posteriore Oberfläche einen Stufe der Brechkraft am äußeren Rand des ersten Bereichs aufweist, oder

wobei die Linse eine anteriore Oberfläche und eine posteriore Oberfläche aufweist, wobei das Brechkraftprofil durch eine Änderung der Oberflächenkrümmung in mindestens einer der anterioren Oberfläche und der posterioren Oberfläche erreicht wird und wobei weder die anteriore Oberfläche noch die posteriore Oberfläche eine Stufe der Brechkraft am äußeren Rand des ersten Bereichs aufweist, oder
wobei die Linse eine anteriore Oberfläche und eine posteriore Oberfläche aufweist, wobei das Brechkraftprofil durch eine Änderung der Oberflächenkrümmung in mindestens einer der anterioren Oberfläche und der posterioren Oberfläche erreicht wird, und wobei der nicht zunehmende Abschnitt des zweiten Bereichs durch eine Brechkraft als Funktion des Radius gekennzeichnet ist, die sich gleichmäßig so ändert, dass der Abstand vom äußeren Rand des ersten Bereichs zu einer radialen Position der minimalen Linsenbrechkraft weniger als 0,4 mm beträgt.

14. Linse nach Anspruch 10, wobei die Linse eine Gradientenindexlinse mit einem Brechungsindex ist, der radial variiert, um das Brechkraftprofil bereitzustellen, und/oder wobei im Wesentlichen die Basisbrechkraft im zweiten Bereich über einen radialen Abstand von mindestens 0,6 mm aufrechterhalten wird, und/oder wobei die Linse ferner eine Oberfläche aufweist, die ein darauf überlagertes diffraktives Profil aufweist.

15. Linse nach Anspruch 14, wobei das diffraktive Profil konfiguriert ist, eine Tiefenschärfe mit einem ersten Peak in einer MTF und einem zweiten Peak in einer MTF zu erzeugen, und wobei das Brechkraftprofil die MTF zwischen dem ersten Peak und dem zweiten Peak erhöht, wobei die MTF unter Verwendung des ISO 1-Modellauges spezifiziert ist, und wobei die MTF für eine Raumfrequenz von 50 lp/mm an der Netzhaut, bei 546 nm Licht, für eine Pupille mit 3 mm Durchmesser, wenn die Linse in Kammerwasser mit einem Brechungsindex von 1,336 bei 546 nm eingetaucht ist, spezifiziert ist, und vorzugsweise

wobei das Brechkraftprofil die MTF nur zwischen dem ersten und dem zweiten Peak erhöht, und/oder
wobei das diffraktive Profil ein bifokales diffraktives Profil ist, und/oder

wobei der maximale MTF-Wert gleich oder größer als 0,35 ist, und/oder

wobei der erste Peak in der MTF und der zweite Peak durch etwa 2,5 Dioptrien voneinander getrennt sind und das Brechkraftprofil eine mit dem ersten Peak kontinuierliche Tiefenschärfe bereitstellt, die eine MTF von 0,15 oder größer für mindestens etwa 1,25 Dioptrien in myopischer Richtung vom ersten Peak aufrechterhält.

**Revendications**

1. Lentille intraoculaire avec une profondeur de champ étendue, la lentille ayant un axe optique et une puissance de base pour obtenir une vision de loin, la lentille comprenant : une optique ayant une surface avec un profil de surface sagittal défini en partie par des caractéristiques EDOF ayant une première région de puissance croissante en fonction de la position radiale et dont la courbure ne diminue pas en fonction de l'augmentation de la position radiale depuis l'axe optique vers un bord extérieur de la première région afin d'obtenir des puissances supérieures à la puissance de base au niveau du bord extérieur, la puissance croissante étant suffisante pour obtenir une profondeur de champ s'étendant de la vision de loin à la vision intermédiaire de 1 dioptrie à 1,5 dioptrie, et le profil de surface sagittal ayant une deuxième région s'étendant radialement vers l'extérieur à partir du bord extérieur qui est non croissante en courbure en fonction de la position radiale croissante à partir du bord extérieur pour obtenir des puissances inférieures à la puissance de base, définissant ainsi une partie non croissante, les courbures dans la deuxième région ne diminuant alors pas pour atteindre la puissance de base, puis maintenant sensiblement la puissance de base sur au moins une distance radiale d'au moins 0,3 mm.

2. Lentille selon la revendication 1, dans laquelle la première région présente une courbure croissante en fonction de l'augmentation de la position radiale depuis l'axe optique vers le bord extérieur de la première région, la deuxième région présente une courbure décroissante en fonction de l'augmentation de la position radiale depuis le bord extérieur pour atteindre des puissances inférieures à la puissance de base, puis une courbure croissante en fonction de la position radiale pour atteindre la puissance de base, et/ou dans laquelle la puissance de base est sensiblement maintenue dans la deuxième région sur une distance radiale d'au moins 0,6 mm.

3. Lentille selon la revendication 1, dans laquelle le profil de surface sagittal peut être spécifié par une équation z(r),

$$\text{où } z(r) = \left\{ R + \sqrt{R^2 - (1+c)r^2} \right\}^{-1} + \alpha_m(r) * r^m \, ,$$

et

où m est égal à 4 ou plus,
r est la distance radiale par rapport à l'axe optique de la lentille,
R est le rayon de courbure de la surface,
c est la constante conique de la surface,
$\alpha_1, \alpha_2$ et A sont des constantes définissant la variation du coefficient du quatrième ordre en fonction de r,
$r_{lim}$ est l'emplacement du bord extérieur de la première région et définit un emplacement de transition radiale pour le coefficient du quatrième ordre ($\alpha(r)$), et de préférence
où m = 4, et de préférence
où $\alpha_m(r)$ varie de manière exponentielle en fonction de la distance radiale, et de préférence où

$$\alpha_m(r) = \begin{cases} r \le r_{lim}; & \alpha_1 + 2(\alpha_2 - \alpha_1)\{(1 + e^{-Ar})^{-1} - 0{,}5\} \\ r > r_{lim}; & \text{le coefficient } \alpha_m \text{ est défini pour atteindre une valeur } \alpha_1 \end{cases}$$

r est la distance radiale par rapport à l'axe optique de la lentille,
R est le rayon de courbure de la surface,
c est la constante conique de la surface,
$\alpha_1, \alpha_2$ et A sont des constantes définissant la variation du coefficient du quatrième ordre en fonction de r,
$r_{lim}$ est l'emplacement du bord extérieur de la première région et définit un emplacement de transition radiale pour le coefficient du quatrième ordre ($\alpha(r)$).

4. Lentille selon la revendication 3, dans laquelle la puissance de base est sensiblement maintenue dans la deuxième région sur une distance radiale d'au moins 0,6 mm, et/ou dans laquelle, pour r > $r_{lim}$

$\alpha(r) = \alpha_1$ , de sorte que $\alpha(r)$ atteint la valeur $\alpha_1$ à $r_{lim}$ , et/ou dans laquelle pour r > $r_{lim}$

$$\alpha(r) = \alpha_2 + 2(\alpha_1 - \alpha_2)\left\{\left(1 + e^{-B(r - r_{lim})}\right)^{-1} - 0{,}5\right\}$$

où B est une constante,
de sorte que $\alpha(r)$ atteint progressivement la valeur $\alpha_1$ pour des valeurs de r supérieures à $r_{lim}$ , et/ou

où z(r) comprend en outre un terme $z_{shift}$
où

$$z_{shift} = \begin{cases} r \leq r_{lim}; & 0 \\ r > r_{lim}; & (\alpha_{lim}(r_{lim})^4 - \alpha_1(r_{lim})^4)\left(2\left(\left(1 + e^{-B(r - r_{lim})}\right)^{-1} - 0{,}5\right)\right) \end{cases}$$

5.  Lentille selon la revendication 1, dans laquelle le profil de surface sagittal peut être spécifié par une équation z(r), où

$$z(r) = r^2\left\{R + \sqrt{R^2 - (1 + c)r^2}\right\}^{-1} + \text{EDOF présente un terme de profil sagittal}$$

et dans laquelle le terme de profil sagittal des caractéristiques EDOF peut être spécifié à l'aide d'une expression polynomiale, et de préférence

dans laquelle l'expression polynomiale approxime une variation exponentielle, et/ou

dans laquelle l'expression polynomiale est de la forme $\sum_{n=1}^{m} C_{2n} r^{2n}$ , et/ou

dans laquelle l'expression polynomiale est du 24e ordre ou supérieur, où m ≥ 12.

6.  Lentille selon la revendication 1, dans laquelle la surface est une surface antérieure ou une surface postérieure, et dans laquelle la partie à courbure non croissante est **caractérisée par** une discontinuité située au niveau du bord extérieur, de telle sorte que le bord extérieur et l'emplacement de la puissance minimale de la lentille coïncident sensiblement radialement.

7.  Lentille selon la revendication 1, dans laquelle la surface est une surface antérieure ou une surface postérieure, et dans laquelle la partie à courbure non croissante varie de manière régulière en fonction du rayon, de telle sorte que la distance entre le bord extérieur de la première région et un emplacement radial de puissance minimale de lentille est inférieure à 0,4 mm, et/ou dans laquelle la lentille est monofocale, et/ou dans laquelle le profil de surface sagittal est en outre défini par un profil diffractif superposé à la surface.

8.  Lentille selon la revendication 7, dans laquelle le profil diffractif est configuré pour produire une profondeur de champ ayant un premier pic dans la MTF et un deuxième pic dans la MTF, et dans laquelle les caractéristiques EDOF augmentent la MTF entre le premier pic et le deuxième pic, dans laquelle la MTF est spécifiée à l'aide du modèle d'œil ISO 1, et dans laquelle la MTF est spécifiée pour une fréquence spatiale de 50 lp/mm au niveau de la rétine, à une lumière de 546 nm, pour une pupille de 3 mm de diamètre, et lorsque la lentille est immergée dans une humeur aqueuse ayant un indice de réfraction de 1,336 à 546 nm, et de préférence

dans laquelle les caractéristiques EDOF augmentent la MTF uniquement entre le premier pic et le deuxième pic, et/ou

dans laquelle le profil diffractif est un profil diffractif bifocal, et/ou dans laquelle la valeur MTF maximale est égale ou supérieure à 0,35.

9.  Lentille selon la revendication 8, dans laquelle le premier pic dans la MTF et le deuxième pic sont séparés d'environ 2,5 dioptries, et les caractéristiques EDOF fournissent une profondeur de champ continue avec le premier pic qui maintient une MTF de 0,15 ou plus pendant au moins environ 1,25 dioptrie, dans la direction myopique à partir du premier pic.

10. Lentille intraoculaire avec une profondeur de champ étendue, la lentille ayant un axe optique et une puissance de base pour obtenir une vision de loin, la lentille comprenant : une optique **caractérisée par** un profil de puissance qui, dans une première région de puissance croissante en fonction de la position radiale, est non décroissant en fonction de l'augmentation de la position radiale depuis l'axe optique vers un bord extérieur de la première région afin d'obtenir une puissance de lentille supérieure à la puissance de base au niveau du bord extérieur, la puissance croissante étant suffisante pour obtenir une profondeur de champ s'étendant de la vision de loin à la vision intermédiaire de 1 dioptrie à 1,5 dioptrie et, dans une deuxième région s'étendant radialement vers l'extérieur à partir du bord extérieur, le profil de puissance n'augmente pas en fonction de l'augmentation de la position radiale à partir du bord extérieur pour obtenir une puissance minimale de la lentille qui est inférieure à la puissance de base, définissant ainsi une partie non croissante, puis non décroissante en fonction de l'augmentation de la position radiale pour atteindre la puissance de base, la deuxième région maintenant alors sensiblement la puissance de base sur au moins une distance radiale d'au moins 0,3 mm.

11. Lentille selon la revendication 10, dans laquelle la première région augmente en puissance en fonction de l'augmentation de la position radiale depuis l'axe optique vers le bord extérieur de la première région, la deuxième région diminue en puissance en fonction de l'augmentation de la position radiale depuis le bord extérieur pour atteindre la puissance minimale de lentille, puis augmente en courbure en fonction de la position radiale pour atteindre la puissance de base, et/ou

   dans laquelle l'augmentation de la puissance dans la première région est comprise entre 0,5 et 5,0 dioptries, et de préférence
   dans laquelle la lentille atteint une profondeur de champ perçue comprise entre 1,0 et 1,5 dioptrie.

12. Lentille selon la revendication 10, dans laquelle la lentille est monofocale, et/ou

   dans laquelle il y a un gradin de puissance au niveau du bord extérieur, et/ou
   dans laquelle la puissance entre le bord extérieur et la puissance minimale de la lentille varie de manière régulière et la diminution de puissance entre le bord extérieur et la puissance minimale de lentille se produit sur une distance radiale inférieure à 0,4 mm.

13. Lentille selon la revendication 10, dans laquelle la lentille comprend une surface antérieure et une surface postérieure, dans laquelle le profil de puissance est obtenu par une modification de la courbure de surface dans au moins l'une de la surface antérieure et de la surface postérieure, et dans laquelle la surface antérieure ou la surface postérieure présente un gradin de puissance au niveau du bord extérieur de la première région, ou

   dans laquelle la lentille comprend une surface antérieure et une surface postérieure, dans laquelle le profil de puissance est obtenu par une modification de la courbure de surface dans au moins l'une de la surface antérieure et de la surface postérieure, et dans laquelle ni la surface antérieure ni la surface postérieure ne présentent de gradin de puissance au niveau du bord extérieur de la première région, ou
   dans laquelle la lentille comprend une surface antérieure et une surface postérieure, dans laquelle le profil de puissance est obtenu par une modification de la courbure de surface dans au moins l'une de la surface antérieure et de la surface postérieure, et dans laquelle la partie non croissante de la deuxième région est **caractérisée par** une puissance en fonction du rayon qui varie de manière régulière de telle sorte que la distance entre le bord extérieur de la première région et un emplacement radial de la puissance minimale de la lentille est inférieure à 0,4 mm.

14. Lentille selon la revendication 10, dans laquelle la lentille est une lentille à gradient d'indice ayant un indice de réfraction qui varie radialement pour fournir le profil de puissance et/ou

   dans laquelle la puissance de base est sensiblement maintenue dans la deuxième région sur une distance radiale d'au moins 0,6 mm, et/ou
   dans laquelle la lentille comprend en outre une surface sur laquelle est superposé un profil diffractif.

15. Lentille selon la revendication 14, dans laquelle le profil diffractif est configuré pour produire une profondeur de champ ayant un premier pic dans la MTF et un deuxième pic dans la MTF, et dans laquelle le profil de puissance augmente la MTF entre le premier pic et le deuxième pic, dans laquelle la MTF est spécifiée à l'aide du modèle d'œil ISO 1, et dans laquelle la MTF est spécifiée pour une fréquence spatiale de 50 lp/mm au niveau de la rétine, à une lumière de 546 nm, pour une pupille de 3 mm de diamètre, lorsque la lentille est immergée dans une humeur aqueuse ayant un indice de réfraction de 1,336 à 546 nm, et de préférence

dans laquelle le profil de puissance augmente la MTF uniquement entre le premier pic et le deuxième pic, et/ou

dans laquelle le profil diffractif est un profil diffractif bifocal, et/ou

dans laquelle la valeur MTF maximale est égale ou supérieure à 0,35, et/ou

dans laquelle le premier pic dans la MTF et le deuxième pic sont séparés d'environ 2,5 dioptries, et le profil de puissance fournit une profondeur de champ continue avec le premier pic qui maintient une MTF de 0,15 ou plus pendant au moins environ 1,25 dioptrie, dans la direction myopique à partir du premier pic.

Fig. 1B

Fig. 1A

**Fig. 2A**

EP 4 305 488 B1

Fig. 2B

Fig. 3

Through-Focus MTF (at 50lp/mm)

Fig. 4

EP 4 305 488 B1

EP 4 305 488 B1

Fig. 5

Fig. 6A

EP 4 305 488 B1

Fig. 6B

Fig. 7

EP 4 305 488 B1

Fig. 8

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2020253719 A1